# EUROPEAN PATENT SPECIFICATION

(11) **EP 4 153 192 B1**
(45) Date of publication and mention of the grant of the patent: **24.07.2024**
(21) Application number: 21725557.9
(22) Date of filing: 19.05.2021
(51) Int. Cl.: A61K 33/26, A61K 39/395, A61P 29/00, A61P 37/02, A61P 39/04, C07K 16/28

(54) **ANTAGONIST OF CD44/HYALURONIC ACID PATHWAY FOR USE IN A METHOD FOR THE TREATMENT OF CYTOKINE RELEASE SYNDROME**
ANTAGONIST DES CD44/HYALURONSÄURE-WEGS ZUR VERWENDUNG IN EINEM VERFAHREN ZUR BEHANDLUNG DES ZYTOKINFREISETZUNGSSYNDROMS
ANTAGONISTE DE LA VOIE CD44/ACIDE HYALURONIQUE POUR UTILISATION DANS UNE MÉTHODE DE TRAITEMENT DU SYNDROME DE LIBÉRATION DE CYTOKINES

(30) Priority: 19.05.2020 EP 20305520
(43) Date of publication of application: 29.03.2023
(73) Proprietor: Institut Curie, 75248 Paris Cedex 05 (FR); Institut National de la Santé et de la Recherche Médicale (INSERM), 75013 Paris (FR); Centre National de la Recherche Scientifique, 75016 Paris (FR)
(72) Inventor: RODRIGUEZ, Raphaël, 75004 PARIS (FR); MULLER, Sebastian, 94200 IVRY-SUR-SEINE (FR); SOLIER, Stéphanie, 75005 PARIS (FR); WATSON, Sarah, 75020 PARIS (FR)
(74) Representative: Cabinet Becker et Associés
(86) International application number: PCT/EP2021/063224
(87) International publication number: WO 2021/233962

(56) References cited:
- CARLO PERRICONE ET AL: "COVID-19 as part of the hyperferritinemic syndromes: is there a role for iron depletion therapy?", AUTHOREA, INC., 6 May 2020 (2020-05-06), XP055747429, Retrieved from the Internet <URL:https://d197for5662m48.cloudfront.net/documents/publicationstatus/35416/preprint_pdf/91503dd383420308c403046292845e4c.pdf> DOI: 10.22541/au.158880283.34604328
- DALAMAGA MARIA ET AL: "Commentary: Could iron chelators prove to be useful as an adjunct to COVID-19 Treatment Regimens?", METABOLISM, CLINICAL AND EXPERIMENTAL, W.B. SAUNDERS CO., PHILADELPHIA, PA, US, vol. 108, 8 May 2020 (2020-05-08), XP086199072, ISSN: 0026-0495, [retrieved on 20200508], DOI: 10.1016/J.METABOL.2020.154260
- SEBASTIAN MÜLLER ET AL: "CD44 regulates epigenetic plasticity by mediating iron endocytosis", BIORXIV, 9 July 2019 (2019-07-09), XP055747098, Retrieved from the Internet <URL:https://www.biorxiv.org/content/10.1101/693424v1.full.pdf> [retrieved on 20201104], DOI: 10.1101/693424
- HERBERT A. RUNNELS ET AL: "PF-03475952: a potent and neutralizing fully human anti-CD44 antibody for therapeutic applications in inflammatory diseases", ADVANCES IN THERAPY., vol. 27, no. 3, 1 March 2010 (2010-03-01), US, pages 168 - 180, XP055747387, ISSN: 0741-238X, DOI: 10.1007/s12325-010-0010-0
- SHI YUFANG ET AL: "COVID-19 infection: the perspectives on immune responses", CELL DEATH & DIFFERENTIATION, NATURE PUBLISHING GROUP, GB, vol. 27, no. 5, 23 March 2020 (2020-03-23), pages 1451 - 1454, XP037098196, ISSN: 1350-9047, [retrieved on 20200323], DOI: 10.1038/S41418-020-0530-3

## Description

### FIELD OF THE INVENTION:

The invention relates to methods and pharmaceutical compositions for the treatment of Cytokine Release Syndrome (CRS). The invention also relates to methods for the diagnosis of patients suffering from Cytokine Release Syndrome.

### BACKGROUND OF THE INVENTION:

Recent work has shown that the cytokine release syndrome (CRS) is the major cause of morbidity and mortality in SARS-CoV-2 patients, where elevated Interleukin 6 (IL6) correlates with respiratory failure (Moore JB, June CH. Cytokine release syndrome in severe COVID-19. Science. 2020 May 1;368(6490):473-474. doi: 10.1126/science.abb8925). It has also been shown that CD44/Hyaluronate (HA) regulate chemokine gene expression in alveolar macrophages (McKee et al., Hyaluronan (HA) fragments induce chemokine gene expression in alveolar macrophages. The role of HA size and CD44. J Clin Invest. 1996 Nov 15;98(10):2403-13. DOI: 10.1172/JCI119054). Furthermore, levels of ferritin have been shown to be highly upregulated in SARS-CoV-2 patients with severe symptoms (Zhou et al., Clinical course and risk factors for mortality of adult inpatients with COVID-19 in Wuhan, China, a retrospective cohort study. The Lancet (2020) DOI: https://doi.org/10.1016/50140-6736(20)30566-3), suggesting a causal role of iron in Covid-19 physiopathology.

Perricone et al (Authorea, Inc, DOI: 10.22541/au.158880283.3460 4328) relates to the role for iron depletion therapy in the context of COVID-19 as part of the hyperferritinemic syndromes. Dalamaga et al (2020, Metabolism, Clinical and experimental, 108, 154260) relates to the possible usefulness of iron chelators as an adjunct to COVID-19 treatment regimens.

The inventors have recently unravelled the functional role of CD44, a plasma membrane receptor of Hyal involved in the regulation of epithelial-mesenchymal transitions, cancer progression, immune responses and inflammation. Specifically, the inventors have discovered that CD44 mediates endocytosis of iron-bound Hyal in cancer cells and immune T-cells. In this context, the inventors have shown that iron plays a central role in the regulation of epigenetic plasticity and cellular identity, acting as a metal catalyst to promote the demethylation of repressive histone marks H3K9me2 and H3K27me3. During the course of this study, the inventors found that the gene coding for IL6 receptor (II,6R/CD126) is one of the top iron-regulated genes, where iron - and thus CD44/Hyal - mediates depletion of H3K9me2 leading to upregulation of IL6R. Importantly, the inventors have also shown that anti-CD44 antibodies can block iron endocytosis, antagonizing this effect. This work has raised considerable interest internationally in the broad scientific community including basic research scientists and clinicians alike (Müller et al., CD44 regulates epigenetic plasticity by mediating iron endocytosis, BioRxiv, DOI: https://doi.org/10.1101/693424, accepted in Nature Chemistry)*.*

Here the inventors demonstrate that during activation of M1 macrophages, iron endocytosis is upregulated in a CD44-dependent manner and that CD44 protein levels increase. This effect is specific to M1 macrophages, whereas levels of the canonical iron endocytosis protein TfR1/CD71 remain unchanged. Altogether, these data are hinting towards a direct role of CD44-mediated iron endocytosis in the severe inflammatory response observed in SARS-CoV-2 patients.

There is also no disclosure in the art of the use of antagonists of the CD44/Hyaluronic Acid (HA) pathway in the treatment of Cytokine Release Syndrome (CRS), particularly in SARS-CoV-2 patients.

### SUMMARY OF THE INVENTION:

The invention relates to methods and pharmaceutical compositions for the treatment of Cytokine Release Syndrome (CRS). The invention also relates to methods for the diagnosis of patients suffering from Cytokine Release Syndrome (CRS). In particular, the invention is defined by the claims.

In an aspect of the disclosure, the present invention relates to an antagonist of CD44/Hyaluronic Acid pathway or a pharmaceutical composition comprising it for use in the treatment of cytokine release syndrome in a subject in need thereof, wherein said antagonist of CD44/Hyaluronic Acid (HA) pathway is selected from the group consisting of a compound targeting CD44 selected from the group consisting of CD44 antagonists and CD44 expression inhibitors. It further relates to the use of a compound targeting CD44 selected from the group consisting of CD44 antagonists and CD44 expression inhibitors for the manufacture of a medicine for the treatment of cytokine release syndrome in a subject in need thereof. It also relates to a method for the treatment of cytokine release syndrome in a subject in need thereof comprising administrating a therapeutic effective amount of a compound targeting CD44 selected from the group consisting of CD44 antagonists and CD44 expression inhibitors, thereby preventing or decreasing the cytokine release syndrome.

In a particular aspect, the cytokine release syndrome is severe COVID-19-related cytokine release syndrome.

Optionally, said CD44 antagonist is selected from the group consisting of small organic molecule, polypeptide, aptamer or antibody. For instance, said antibody is selected from the group consisting of RG7356 and Bivatuzumab.

Optionally, said CD44 expression inhibitor is selected from the group consisting of antisense oligonucleotide, shRNA, siRNA, RNAi, and ribozyme.

### DETAILED DESCRIPTION OF THE INVENTION:

The inventors investigate iron homeostasis and the role of CD44-mediated iron endocytosis in the severe inflammatory response and Cytokine Release Syndrome (CRS), particularly in SARS-CoV-2 patients. The inventors demonstrate that during activation of M1 macrophages, iron endocytosis is upregulated in a CD44-dependent manner and that CD44 protein levels increase. This effect is specific to M1 macrophages, whereas levels of the canonical iron endocytosis protein TfR1/CD71 remain unchanged. In the present invention, the inventors provide *in vitro* evidences towards a direct role of CD44-mediated iron endocytosis in the severe inflammatory response such as Cytokine Release Syndrome (CRS) observed in SARS-CoV-2 patients. Altogether, the present invention highlights the role of CD44-mediated iron endocytosis in the severe inflammatory response and Cytokine Release Syndrome (CRS), and the potential use of antagonists of CD44/Hyaluronic Acid (HA) pathway and the targeting of iron homeostasis in the treatment of Cytokine Release Syndrome (CRS), particularly in SARS-CoV-2 patients. The invention also identifies CD44 as a new marker of predisposition to severe COVID-19.

Accordingly, the present disclosure relates to the targeting of iron homeostasis in the treatment of Cytokine Release Syndrome (CRS), particularly severe COVID-19-related CRS. The invention also relates to the targeting of iron homeostasis in the treatment of Adult Respiratory Distress Syndrome (ARDS), particularly Severe COVID-19-related ARDS. The invention also relates to the targeting of iron homeostasis in the treatment of Macrophage Activation Syndrome (MAS), particularly Severe COVID-19-related MAS. The invention also relates to the targeting of iron homeostasis in the treatment of Iron related inflammatory diseases and Alveolar inflammatory responses, particularly Iron related inflammatory diseases and Alveolar inflammatory responses in COVID-19 patients. The invention also relates to the targeting of iron homeostasis in the treatment of COVID-19, particularly Severe COVID-19.

### Therapeutic method

In a first aspect, the invention relates to an antagonist of CD44/Hyaluronic Acid (HA) pathway for use in the treatment of Cytokine Release Syndrome (CRS) in a subject in need thereof.

In some embodiments, the invention relates to an antagonist of CD44/Hyaluronic Acid (HA) pathway for use in the treatment of severe COVID-19-related CRS in a subject in need thereof.

In a further aspect, the invention relates to an antagonist of CD44/Hyaluronic Acid (HA) pathway for use in the treatment of Respiratory Distress Syndrome (ARDS) in a subject in need thereof.

In a further aspect, the invention relates to an antagonist of CD44/Hyaluronic Acid (HA) pathway for use in the treatment of Macrophage Activation Syndrome (MAS) in a subject in need thereof.

In a further aspect, the invention relates to an antagonist of CD44/Hyaluronic Acid (HA) pathway for use in the treatment of Iron related inflammatory diseases and Alveolar inflammatory responses in a subject in need thereof.

In a further aspect, the invention relates to an antagonist of CD44/Hyaluronic Acid (HA) pathway for use in the treatment of COVID-19, particularly Severe COVID-19 in a subject in need thereof.

As used herein, the terms "subject", "individual" or "patient" are interchangeable and refer to a mammal. Typically, a subject according to the invention refers to any subject, preferably human. In a particular embodiment, the term "subject" refers to a subject afflicted or at risk to be afflicted with Cytokine Release Syndrome (CRS), particularly COVID-19-related CRS. In a particular embodiment, the term "subject" refers to a subject afflicted or at risk to be afflicted with Adult Respiratory Distress Syndrome (ARDS), particularly Severe COVID-19-related ARDS. In a particular embodiment, the term "subject" refers to a subject afflicted or at risk to be afflicted with Macrophage Activation Syndrome (MAS), particularly Severe COVID-19-related MAS. In a particular embodiment, the term "subject" refers to a subject afflicted or at risk to be afflicted with Iron related inflammatory diseases and Alveolar inflammatory responses, particularly Iron related inflammatory diseases and Alveolar inflammatory responses in COVID-19 patients. In a particular embodiment, the term "subject" refers to a subject afflicted or at risk to be afflicted with COVID-19, particularly Severe COVID-19.

As used herein, the term "treatment" or "treat" refer to both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "Cytokine Release Syndrome" or "CRS" has its general meaning in the art and refers to a disease caused by a large and rapid release of cytokines into the blood from immune cells. The term "Cytokine Release Syndrome" or "CRS" also refers to a systemic inflammatory response observed following administration of antibodies, and adoptive T cell therapy or a cytokine reactions occurring in infectious disease such as coronavirus disease 2019 (COVID-19), sepsis, Ebola, avian influenza and non-infectious diseases such as graft-versus-host disease (GVHD), acute respiratory distress syndrome (ARDS) and systemic inflammatory response syndrome (SIRS) (Shimabukuro-Vornhagen et al., Cytokine release syndrome. J Immunother Cancer. 2018 Jun 15;6(1):56. doi:10.1186/s40425-018-0343-9). The term "Cytokine Release Syndrome" or "CRS" refers such as described in the National Cancer Institute (NCI) Dictionary of Cancer Terms to a condition that may occur after treatment with some types of immunotherapy, such as antibodies and CAR-T cells and caused by a large and rapid release of cytokines into the blood from immune cells affected by the immunotherapy. CRS is the most common acute toxicity of CAR T-cells, CRS is related to a progressive systemic inflammatory process and is characterized by high fever, hypotension, hypoxia, neurologic toxicities and/or multiorgan toxicity (Davila et al., Efficacy and toxicity management of 19-28z CAR T cell therapy in B cell acute lymphoblastic leukemia. Sci Transl Med. 2014 Feb 19;6(224):224ra25. doi: 10.1126/scitranslmed). Severity of CRS can vary from mild symptoms including fever, myalgia, and fatigue, to severe symptoms including but not limited to acute respiratory distress syndrome (ARDS), hypotension, disseminated intravascular coagulation, renal and liver toxicities, organ dysfunction, an increase in cytokine profiles such as IL6, IL2R, IL-1 beta and/or neurotoxicity (Shalabi et al., Novel Designs of Early Phase Trials for Cancer Therapeutics. Chapter 12 - Cell-Based Therapies: A New Frontier of Personalized Medicine. Editor(s): Shivaani Kummar, Chris Takimoto. Academic Press, 2018, Pages 175-191, ISBN 9780128125120, doi.org/10.1016/B978-0-12-812512-0.00012-9; Maude et al., Managing cytokine release syndrome associated with novel T cell-engaging therapies. Cancer J. 2014 Mar-Apr;20(2):119-22. doi: 10.1097/PPO.0000000000000035). Cytokine Release Syndrome may also be associated with findings of macrophage activation syndrome (MAS)/hemophagocytic lymphohistiocytosis (HLH). The term "Cytokine Release Syndrome" also refers to COVID-related CRS, particularly severe COVID-19-related CRS. Recent work has shown that the cytokine release syndrome (CRS) is the major cause of morbidity and mortality in SARS-CoV-2 patients, where elevated IL6 correlates with respiratory failure (Moore JB, June CH. Cytokine release syndrome in severe COVID-19. Science. 2020 May 1;368(6490):473-474. doi: 10.1126/science.abb8925).

As used herein, the term "Adult Respiratory Distress Syndrome" or "ARDS" has its general meaning in the art and refers to an acute and intense pulmonary inflammatory process caused by pulmonary or systemic insults, most commonly initiated by pneumonia, infectious diseases, sepsis or trauma (Ranieri et al., ARDS Definition Task Force. Acute respiratory distress syndrome: the Berlin Definition. JAMA. 2012 Jun 20;307(23):2526-33. doi:10.1001/jama.2012.5669; Hernández-Beeftink et al., Genomics and the Acute Respiratory Distress Syndrome: Current and Future Directions. Int J Mol Sci. 2019 Aug 16;20(16). pii: E4004. doi: 10.3390/ijms20164004). The term "Adult Respiratory Distress Syndrome" or "ARDS" also refers to a life-threatening lung injury that allows fluid to leak into the lungs, breathing becomes difficult and oxygen cannot get into the body such as described by The American Lung Association (lung.org/lung-health-diseases). The term "Adult Respiratory Distress Syndrome" or "ARDS" also refers to a respiratory failure characterized by rapid onset of extensive inflammation in the lungs with signs and symptoms such as severe shortness of breath, labored and unusually rapid breathing, low blood pressure, confusion and extreme tiredness, as well as the presence of underlying heart or lung disease such as described in the Mayo Clinic College of Medicine and Science (mayoclinic.org/patient-care-and-health-information). The most common underlying causes of ARDS include infectious disease, sepsis, inhalation of harmful substances, pancreatitis, trauma, pneumonia, massive blood transfusions and burns. The pathophysiology of ARDS results from acute inflammation in the alveolar space and decrease in normal gas exchange. Fibrin deposition in the air spaces and lung parenchyma, are consistently observed with ARDS and contributes to hyaline-membrane formation and subsequent alveolar fibrosis. This promotes the development and progression of respiratory dysfunction and right heart failure (Whyte CS, Morrow GB, Mitchell JL, Chowdary P, Mutch NJ. Fibrinolytic abnormalities in acute respiratory distress syndrome (ARDS) and versatility of thrombolytic drugs to treat COVID-19. J Thromb Haemost. 2020 Apr 23. doi:10.1111/jth.14872). The term "Adult Respiratory Distress Syndrome" or "ARDS" also refers to Severe COVID-19-related ARDS.

As used herein, the term "Macrophage Activation Syndrome" or "MAS" has its general meaning in the art and refers to a disease characterized by pancytopenia, liver insufficiency, coagulopathy, and neurologic symptoms and is thought to be caused by the activation and uncontrolled proliferation of T lymphocytes and well-differentiated macrophages, leading to widespread hemophagocytosis and cytokine overproduction. The term "Macrophage Activation Syndrome" or "MAS" also refers to a life-threatening complication of rheumatic disease that occurs much more frequently in individuals with systemic juvenile idiopathic arthritis (SJIA) and in those with adult-onset Still disease (Crayne et al., The Immunology of Macrophage Activation Syndrome. Front Immunol. 2019 Feb 1;10:119. doi:10.3389/fimmu.2019.00119; Lerkvaleekul and Vilaiyuk. Macrophage activation syndrome: early diagnosis is key. Open Access Rheumatol. 2018 Aug 31;10:117-128. doi: 10.2147/OARRR.S151013). The term "Macrophage Activation Syndrome" also refers to Severe COVID-19-related MAS (Merad and Martin. Pathological inflammation in patients with COVID-19: a key role for monocytes and macrophages. Nat. Rev. Immunol. 2020 May 6. doi: 10.103 8/s41577-020-0331-4).

As used herein, the term "Iron related inflammatory diseases" has its general meaning in the art and refers to inflammatory diseases related to dysfunction in iron homeostasis. In some embodiments, the term "Iron related inflammatory diseases" relates to inflammatory diseases related to Iron Overload Syndromes. The term "Iron related inflammatory diseases" also refers to Iron related severe inflammatory response, particularly Iron related severe inflammatory response in COVID-19 patients.

As used herein, the term "Alveolar inflammatory responses" has its general meaning in the art and refers to inflammatory diseases related to dysfunction of alveolar macrophage function such as after chest trauma, ischemia/reperfusion, hemorrhagic shock, and thermal burns. The term "Alveolar inflammatory responses" also refers to inflammatory diseases related to enhanced chemokine gene expression in alveolar macrophages associated with Acute Lung Injury (ALI) and Acute Respiratory Distress Syndrome (ARDS) (Niesler et al., Role of alveolar macrophages in the inflammatory response after trauma. Shock. 2014 Jul;42(1):3-10. doi: 10.1097/SHK.0000000000000167). The term "Alveolar inflammatory responses" also refers to COVID-19-related Alveolar inflammatory responses.

As used herein, the term "COVID-19" or "Coronavirus disease 2019" has its general meaning in the art and refers to an infectious coronavirus disease caused by severe acute respiratory syndrome coronavirus 2 (SARS-CoV-2), a newly identified coronavirus in December 2019 in Wuhan, China. The term "COVID-19" also refers to 2019-nCoV acute respiratory disease. COVID-19 results in mild to moderate respiratory disease, but may in some cases develop into severe COVID-19.

As used herein, the term "Severe COVID-19" has its general meaning in the art and refers to COVID-19 side effect resulting in severe respiratory disease, pneumonia, viral sepsis, Cytokine Release Syndrome (CRS), Acute Respiratory Distress Syndrome (ARDS), Macrophage Activation Syndrome (MAS), multi-visceral failure syndrome caused by an enhanced inflammatory response such as kidney and lung failure, respiratory failure, arterial inflammation, myocarditis (also known as inflammatory cardiomyopathy), myocardial injury, thrombosis, venous thromboembolic event, cardiovascular diseases such as described in Han Y, Zeng H, Jiang H, Yang Y, Yuan Z, Cheng X, Jing Z, Liu B, Chen J, Nie S, Zhu J, Li F, Ma C. CSC Expert Consensus on Principles of Clinical Management of Patients with Severe Emergent Cardiovascular Diseases during the COVID-19 Epidemic. Circulation. 2020 Mar 27. doi: 10.1161/CIRCULATIONAHA.120.047011), pulmonary embolism, neurologic toxicities, Kawasaki disease (also known as mucocutaneous lymph node syndrome) and Cutaneous manifestations of COVID-19 such as described in (Sachdeva M, Gianotti R, Shah M, Lucia B, Tosi D, Veraldi S, Ziv M, Leshem E, Dodiuk-Gad RP. Cutaneous manifestations of COVID-19: Report of three cases and a review of literature. J Dermatol Sci. 2020 Apr 29. pii: S0923-1811(20)30149-3. doi: 10.1016/j.jdermsci.2020.04.011).

The term "CD44" has its general meaning in the art and refers to a transmembrane glycoprotein CD44 Molecule (Indian Blood Group) involved in cell-cell interactions, cell adhesion and migration. The term "CD44" as used herein is intended to designate a transmembrane glycoprotein, a plasma membrane receptor of hyaluronic acid (HA or Hyal) involved in a wide variety of physiological and pathological processes including development, inflammation, immune responses, wound healing, regulation of epithelial-mesenchymal transitions (EMT) and cancer progression (Ponta, H., Sherman, L. & Herrlich, P. A. CD44: from adhesion molecules to signalling regulators. Nat. Rev. Mol. Cell Biol. 4, 33-45, (2003); Zöller, M. CD44: can a cancer-initiating cell profit from an abundantly expressed molecule? Nat. Rev. Cancer 11, 254-267, (2011)). The term "CD44" also refers to the cell-surface glycoprotein its isoforms having the GeneCards Identifier GC11P035139 and UniProt Knowledgebase UniProtKB/Swiss-Prot database identifier P16070 (Stelzer et al., The GeneCards Suite: From Gene Data Mining to Disease Genome Sequence Analyses. Curr Protoc Bioinformatics. 2016 Jun 20;54:1.30.1-1.30.33. doi: 10.1002/cpbi.5; UniProt Consortium. UniProt: a worldwide hub of protein knowledge. Nucleic Acids Res. 2019 Jan 8;47(D1):D506-D515. doi: 10.1093/nar/gky1049). CD44 interacts with and mediates endocytosis of hyaluronates (Hyal), a class of glycosaminoglycan biopolymers (Aruffo, A., Stamenkovic, I., Melnick, M., Underhill, C. B. & Seed, B. CD44 is the principal cell surface receptor for hyaluronate. Cell 61, 1303-1313, (1990); Hua, Q., Knudson, C. B. & Knudson, W. Internalization of hyaluronan by chondrocytes occurs via receptor-mediated endocytosis. J. Cell Sci. 106, 365-375, (1993)).

The term "Hyaluronic Acid" or "HA" or "Hyal" has its general meaning in the art and refers to Hyaluronic acid (HA) also known as hyaluronate (Hyal) and hyaluronan. The term "Hyaluronic Acid" or "HA" as used herein is intended to designate a class of glycosaminoglycan biopolymers involved in a wide variety of cellular functions including cell proliferation, cell migration, epithelial-mesenchymal transitions (EMT) and cancer progression (Aruffo, A., Stamenkovic, I., Melnick, M., Underhill, C. B. & Seed, B. CD44 is the principal cell surface receptor for hyaluronate. Cell 61, 1303-1313, (1990); Hua, Q., Knudson, C. B. & Knudson, W. Internalization of hyaluronan by chondrocytes occurs via receptor-mediated endocytosis. J. Cell Sci. 106, 365-375, (1993)).

As used herein, the term "antagonist of CD44/Hyaluronic Acid (HA) pathway" refers to any compound selected from the group consisting of but not limited to compounds targeting iron homeostasis, compounds targeting CD44 selected from the group consisting of CD44 antagonist or CD44 expression inhibitor, compounds targeting Hyaluronic Acid (HA), inhibitors of Hyaluronic Acid (HA)/Iron internalization, inhibitors of iron endocytosis, Iron chelators such as deferoxamine (DFO); deferoxamine mesylate; deferoxamine hydrochloride; deferasirox (DFX); deferiprone (DFP); deferitrin; deferipone; metformin; metforminyn; deferitazole; hepcidin fragments; and siderophores. The antagonist of CD44/Hyaluronic Acid (HA) pathway has its therapeutic effect by antagonizing IL6 signalling.

The term "CD44 antagonist" has its general meaning in the art and refers to a compound that selectively inactivates the CD44. The term "CD44 antagonist" refers to any compound that can directly or indirectly inhibit the signal transduction cascade related to CD44 and inhibits CD44 ligand binding (Hyaluronic Acid (HA)), inhibits iron binding to CD44/HA complex, inhibits endocytosis of iron-bound CD44/HA complex, inhibits iron-bound Hyal internalization, and then inhibits demethylation of repressive histone marks H3K9me2 and H3K27me3, inhibits the depletion of H3K9me2 and inhibits the upregulation of iron-regulated genes (such as IL6 receptor (IL6R/CD126)). As used herein, the term "selectively inactivates" refers to a compound that preferentially inactivates CD44 with a greater affinity and potency, respectively, than its interaction with the other sub-types or isoforms of the CD44 signaling family of receptors and cell-surface glycoprotein. CD44 antagonist also refers to a compound that decreases CD44 activity level. Compounds that prefer CD44, but that may also inactivate other CD44 signaling family of receptors sub-types and cell-surface glycoproteins, as partial or full antagonists are contemplated. Typically, a CD44 antagonist is a small organic molecule, a polypeptide, an aptamer or an antibody.

CD44 antagonists are well-known in the art as such as described in WO2008/144890; WO0139815; WO2004/018000; WO2005/049082; WO2008/079246; WO2014/023869; WO2005/087264.

Tests and assays for determining whether a compound is a CD44 antagonist are well known by the skilled person in the art such as described in WO2014/198843; WO2008/144890; WO2011/095498; WO2013/063498; WO2004/018000; WO2005/049082; WO2008/079246; WO2005/087264; WO2014/023869; WO2007/039761 "CRYSTAL STRUCTURE OF CD44 AND ITS USE"; Weigand *et al.,* 2012; D'Arena *et al.,* 2014.

In some embodiments, the CD44 antagonist is an anti-CD44 antibody selected from but not limited to the group consisting of RG7356 anti-CD44 antibody; Bivatuzumab; and antibodies described in WO2008/144890; WO2004/018000; WO2005/049082; WO2008/079246; WO2005/087264.

In some embodiments, the CD44 antagonist is a peptide or a CD44 ligand selected from but not limited to ligand described in WO2015/097170.

In some embodiments, the antibodies of the present invention compete for binding to CD44 (and Hyaluronic Acid (HA)) antibodies described above.

In another embodiment, the compound of the invention is an aptamer. Aptamers are a class of molecule that represents an alternative to antibodies in term of molecular recognition. Aptamers are oligonucleotide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996). Then after raising aptamers directed against the target of the invention as above described, the skilled man in the art can easily select those blocking or inactivating the target.

In another embodiment, the compound of the invention is an antibody (the term including "antibody portion") directed against the target.

In one embodiment of the antibodies or portions thereof described herein, the antibody is a monoclonal antibody. In one embodiment of the antibodies or portions thereof described herein, the antibody is a polyclonal antibody. In one embodiment of the antibodies or portions thereof described herein, the antibody is a humanized antibody. In one embodiment of the antibodies or portions thereof described herein, the antibody is a chimeric antibody. In one embodiment of the antibodies or portions thereof described herein, the portion of the antibody comprises a light chain of the antibody. In one embodiment of the antibodies or portions thereof described herein, the portion of the antibody comprises a heavy chain of the antibody. In one embodiment of the antibodies or portions thereof described herein, the portion of the antibody comprises a Fab portion of the antibody. In one embodiment of the antibodies or portions thereof described herein, the portion of the antibody comprises a F(ab')2 portion of the antibody. In one embodiment of the antibodies or portions thereof described herein, the portion of the antibody comprises a Fc portion of the antibody. In one embodiment of the antibodies or portions thereof described herein, the portion of the antibody comprises a Fv portion of the antibody. In one embodiment of the antibodies or portions thereof described herein, the portion of the antibody comprises a variable domain of the antibody. In one embodiment of the antibodies or portions thereof described herein, the portion of the antibody comprises one or more CDR domains of the antibody.

As used herein, "antibody" includes both naturally occurring and non-naturally occurring antibodies. Specifically, "antibody" includes polyclonal and monoclonal antibodies, and monovalent and divalent fragments thereof. Furthermore, "antibody" includes chimeric antibodies, wholly synthetic antibodies, single chain antibodies, and fragments thereof. The antibody may be a human or nonhuman antibody. A nonhuman antibody may be humanized by recombinant methods to reduce its immunogenicity in man.

Antibodies are prepared according to conventional methodology. Monoclonal antibodies may be generated using the method of Kohler and Milstein (Nature, 256:495, 1975). To prepare monoclonal antibodies useful in the invention, a mouse or other appropriate host animal is immunized at suitable intervals (e.g., twice-weekly, weekly, twice-monthly or monthly) with antigenic forms of the target. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes.

Briefly, the antigen may be provided as synthetic peptides corresponding to antigenic regions of interest in the target. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope (see, in general, Clark, W. R. (1986) The Experimental Foundations of Modern Immunology Wiley & Sons, Inc., New York; Roitt, I. (1991) Essential Immunology, 7th Ed., Blackwell Scientific Publications, Oxford). The Fc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDRS). The CDRs, and in particular the CDRS regions, and more particularly the heavy chain CDRS, are largely responsible for antibody specificity.

It is now well-established in the art that the non CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody.

This invention provides in certain embodiments compositions and methods that include humanized forms of antibodies. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,859,205, which are hereby incorporated by reference. The above U.S. Pat. Nos. 5,585,089 and 5,693,761, and WO 90/07861 also propose four possible criteria, which may be used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgGl, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., /. Mol. Biol. 294:151, 1999, the contents of which are incorporated herein by reference.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, the contents of which are incorporated herein by reference. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans.

In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610). The contents of these patents are incorporated herein by reference.

Thus, as will be apparent to one of ordinary skill in the art, the present invention also provides for F(ab') 2 Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

The various antibody molecules and fragments may derive from any of the commonly known immunoglobulin classes, including but not limited to IgA, secretory IgA, IgE, IgG and IgM. IgG subclasses are also well known to those in the art and include but are not limited to human IgGl, IgG2, IgG3 and IgG4. In a preferred embodiment, the compound of the invention is a Human IgG4.

In another embodiment, the antibody according to the invention is a single domain antibody. The term "single domain antibody" (sdAb) or "VHH" refers to the single heavy chain variable domain of antibodies of the type that can be found in Camelid mammals, which are naturally devoid of light chains. Such VHH are also called "nanobody^{®}". According to the invention, sdAb can particularly be llama sdAb. The term "VHH" refers to the single heavy chain having 3 complementarity determining regions (CDRs): CDR1, CDR2 and CDR3. The term "complementarity determining region" or "CDR" refers to the hypervariable amino acid sequences which define the binding affinity and specificity of the VHH.

The VHH according to the invention can readily be prepared by an ordinarily skilled artisan using routine experimentation. The VHH variants and modified form thereof may be produced under any known technique in the art such as in-vitro maturation.

VHHs or sdAbs are usually generated by PCR cloning of the V-domain repertoire from blood, lymph node, or spleen cDNA obtained from immunized animals into a phage display vector, such as pHEN2. Antigen-specific VHHs are commonly selected by panning phage libraries on immobilized antigen, e.g., antigen coated onto the plastic surface of a test tube, biotinylated antigens immobilized on streptavidin beads, or membrane proteins expressed on the surface of cells. However, such VHHs often show lower affinities for their antigen than VHHs derived from animals that have received several immunizations. The high affinity of VHHs from immune libraries is attributed to the natural selection of variant VHHs during clonal expansion of B-cells in the lymphoid organs of immunized animals. The affinity of VHHs from non-immune libraries can often be improved by mimicking this strategy in vitro, i.e., by site directed mutagenesis of the CDR regions and further rounds of panning on immobilized antigen under conditions of increased stringency (higher temperature, high or low salt concentration, high or low pH, and low antigen concentrations). VHHs derived from camelid are readily expressed in and purified from the E. coli periplasm at much higher levels than the corresponding domains of conventional antibodies. VHHs generally display high solubility and stability and can also be readily produced in yeast, plant, and mammalian cells. For example, the "Hamers patents" describe methods and techniques for generating VHH against any desired target (see for example US 5,800,988; US 5,874, 541 and US 6,015,695). The "Hamers patents" more particularly describe production of VHHs in bacterial hosts such as E. coli (see for example US 6,765,087) and in lower eukaryotic hosts such as moulds (for example Aspergillus or Trichoderma) or in yeast (for example Saccharomyces, Kluyveromyces, Hansenula or Pichia) (see for example US 6,838,254).

In another aspect, the invention provides an antibody that competes for binding to the target with the antibody of the invention.

As used herein, the term "binding" in the context of the binding of an antibody to a predetermined antigen or epitope typically is a binding with an affinity corresponding to a KD of about 10-7 M or less, such as about 10-8 M or less, such as about 10-9 M or less, about 10-10 M or less, or about 10-11 M or even less when determined by for instance surface plasmon resonance (SPR) technology in a BIAcore 3000 instrument using a soluble form of the antigen as the ligand and the antibody as the analyte. BIACORE^{®} (GE Healthcare, Piscaataway, NJ) is one of a variety of surface plasmon resonance assay formats that are routinely used to epitope bin panels of monoclonal antibodies. Typically, an antibody binds to the predetermined antigen with an affinity corresponding to a KD that is at least ten-fold lower, such as at least 100-fold lower, for instance at least 1,000-fold lower, such as at least 10,000-fold lower, for instance at least 100,000-fold lower than its KD for binding to a non-specific antigen (e.g., BSA, casein), which is not identical or closely related to the predetermined antigen. When the KD of the antibody is very low (that is, the antibody has a high affinity), then the KD with which it binds the antigen is typically at least 10,000-fold lower than its KD for a non-specific antigen. An antibody is said to essentially not bind an antigen or epitope if such binding is either not detectable (using, for example, plasmon resonance (SPR) technology in a BIAcore 3000 instrument using a soluble form of the antigen as the ligand and the antibody as the analyte), or is 100 fold, 500 fold, 1000 fold or more than 1000 fold less than the binding detected by that antibody and an antigen or epitope having a different chemical structure or amino acid sequence.

Additional antibodies can be identified based on their ability to cross-compete (e.g., to competitively inhibit the binding of, in a statistically significant manner) with other antibodies of the invention in standard antigen binding assays. The ability of a test antibody to inhibit the binding of antibodies of the present invention to the target demonstrates that the test antibody can compete with that antibody for binding to the target; such an antibody may, according to non-limiting theory, bind to the same or a related (e.g., a structurally similar or spatially proximal) epitope on the target as the antibody with which it competes. Thus, another aspect of the invention provides antibodies that bind to the same antigen as, and compete with, the antibodies disclosed herein. As used herein, an antibody "competes" for binding when the competing antibody inhibits the target binding of an antibody or antigen binding fragment of the invention by more than 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99% in the presence of an equimolar concentration of competing antibody.

In other embodiments the antibodies or antigen binding fragments of the invention bind to one or more epitopes of the target. In some embodiments, the epitopes to which the present antibodies or antigen binding fragments bind are linear epitopes. In other embodiments, the epitopes to which the present antibodies or antigen binding fragments bind are non-linear, conformational epitopes.

In one embodiment, the antagonist of CD44/Hyaluronic Acid (HA) pathway of the invention is a CD44 expression inhibitor.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs, which are modified, by processes such as capping, polyadenylation, methylation, and editing, and proteins modified by, for example, methylation, acetylation, phosphorylation, ubiquitination, SUMOylation, ADP-ribosylation, myristilation, and glycosylation.

An "inhibitor of expression" refers to a natural or synthetic compound that has a biological effect to inhibit the expression of a gene. An "inhibitor of expression" refers to any compound that has a biological effect to inhibit the expression of a target gene and/or the expression of target protein. In one embodiment of the invention, said inhibitor of expression is a short hairpin RNA (shRNA), a small inhibitory RNA (siRNA), or an antisense oligonucleotide. Preferably, the inhibitor of expression is a siRNA or a shRNA.

The target expression inhibitors for use in the present invention may be based on antisense oligonucleotide constructs. Anti-sense oligonucleotides, including anti-sense RNA molecules and anti-sense DNA molecules, would act to directly block the translation of the target mRNA by binding thereto and thus preventing protein translation or increasing mRNA degradation, thus decreasing the level of the target proteins, and thus activity, in a cell. For example, antisense oligonucleotides of at least about 15 bases and complementary to unique regions of the mRNA transcript sequence encoding the target can be synthesized, e.g., by conventional phosphodiester techniques and administered by e.g., intravenous injection or infusion. Methods for using antisense techniques for specifically alleviating gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as a target expression inhibitors for use in the present invention. The target gene expression can be reduced by contacting the subject or cell with a small double stranded RNA (dsRNA), or a vector or construct causing the production of a small double stranded RNA, such that the target expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Short hairpin RNA (shRNA) or Small inhibitory RNAs (siRNAs) can function as inhibitors of gene expression for use in the invention. Gene expression can be reduced with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known.

Ribozymes can also function as target expression inhibitors for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of the target mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides (ODNs) and ribozymes useful as target inhibitors can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides, siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing the target. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In some embodiments, the CD44 expression inhibitor is a nucleic acid inhibiting CD44 genes such as described in CN104561000.

In another aspect of the disclosure, the antagonist of CD44/Hyaluronic Acid (HA) pathway of the invention is an iron chelator.

As used herein, the term "iron chelator" has its general meaning in the art and refers to any compound chelating iron and then depleting iron cellular level. The term "iron chelator" also refers to are compounds that remove the excess of iron, toxic iron from the patient's blood, tissues and organs.

Iron chelators are well-known in the art as such as described in WO0149266; WO2009103727; WO2011109521; WO2014059417; WO2017153475; WO2019233982 and in Lima et al., Treatment of iron overload syndrome: a general review. Rev Assoc Med Bras (1992). 2019 Oct 10;65(9):1216-1222. doi:10.1590/1806-9282.65.9.1216; Mobarra et al., A Review on Iron Chelators in Treatment of Iron Overload Syndromes. Int J Hematol Oncol Stem Cell Res. 2016 Oct 1;10(4):239-247; Khan et al., Synthesis, nature and utility of universal iron chelator - Siderophore: A review. Microbiol Res. 2018 Jul-Aug;212-213:103-111. doi: 10.1016/j.micres.2017.10.012; Khan et al., Synthesis, nature and utility of universal iron chelator - Siderophore: A review. Microbiol Res. 2018 Jul-Aug;212-213:103-111. doi: 10.1016/j.micres.2017.10.012; Kontoghiorghes GJ. Comparative efficacy and toxicity of desferrioxamine, deferiprone and other iron and aluminium chelating drugs. Toxicol Lett. 1995 Oct;80(1-3):1-18.

Tests and assays for determining whether a compound is an iron chelator are well known by the skilled person in the art such as described in WO0149266; WO2009103727; WO2011109521; WO2014059417; WO2017153475; WO2019233982.

In some aspects, the iron chelator is selected from but not limited to the group consisting of deferoxamine (DFO) (also known as desferal, desferrioxamine, DFB, CAS Number: 70-51-9); deferoxamine mesylate (CAS Number: 138-14-7); deferoxamine hydrochloride (CAS Number: 1950-39-6); deferasirox (DFX); deferiprone (DFP); deferitrin; deferipone; metformin; metforminyn; deferitazole; 3-hydroxy-4-pyridinone; 3-hydroxy-4-pyridinone derivatives; hepcidin and hepcidin fragments (mini-hepcidins); Polyamine-dihydroxybenzoic acid and Polyamine-dihydroxybenzoic acid conjugate; pyridoxal isonicotinoyl hydrazone (PIH); Rhodotorulic acid; N,N'-Bis(2-hydroxybenzyl)ethylenediamine-N,N-diacetic acid (HBED), N,N'-Bis(2-hydroxybenzyl)propylene-1,3-diamine-N,N-diacetic acid (HBPD), 2,3-dihydroxybebzoic acid; diethyltriamine pentaacetic acid (DTPA) and compounds described in US Patent number US5374771; International Patent applications numbers WO0149266; WO2009103727; WO2011109521; WO2014059417; WO2017153475; WO2019233982; and in Lima et al., Treatment of iron overload syndrome: a general review. Rev Assoc Med Bras (1992). 2019 Oct 10;65(9):1216-1222. doi:10.1590/1806-9282.65.9.1216; Kontoghiorghes GJ. Comparative efficacy and toxicity of desferrioxamine, deferiprone and other iron and aluminium chelating drugs. Toxicol Lett. 1995 Oct;80(1-3):1-18.

In some aspects, the iron chelator is a derivative of biguanide selected from but not limited to the group consisting of metformin, metformin derivatives such as metforminyn and compounds comprising biguanidyl radical described in WO2019233982.

In some aspects, the iron chelator is selected from but not limited to the group consisting of iron chelators produced by bacterial siderophores and siderophores (catecholate, hydroxamate, carboxylate or mixed types) such as desferrioxamine; catecholate siderophore: enterobactin; vibrioferrin; mixed siderophore: yersiniabactin; and phytosiderophore: mugineic acid; and iron chelators described in (Khan et al., Synthesis, nature and utility of universal iron chelator - Siderophore: A review. Microbiol Res. 2018 Jul-Aug;212-213:103-111. doi: 10.1016/j.micres.2017.10.012).

In some aspects, the iron chelator is the lysosomal iron-sequestering small molecule ironomycin.

In a further aspect, the invention relates to a method of treating Cytokine Release Syndrome (CRS) in a subject in need thereof, comprising administering to the subject a therapeutically effective amount of an antagonist of CD44/Hyaluronic Acid (HA) pathway.

In some embodiments, the invention relates to a method of treating severe COVID-19-related CRS in a subject in need thereof, comprising administering to the subject a therapeutically effective amount of an antagonist of CD44/Hyaluronic Acid (HA) pathway.

In a further aspect, the invention relates to a method of treating Respiratory Distress Syndrome (ARDS), Macrophage Activation Syndrome (MAS), Iron related inflammatory diseases and/or Alveolar inflammatory responses in a subject in need thereof, comprising administering to the subject a therapeutically effective amount of an antagonist of CD44/Hyaluronic Acid (HA) pathway.

In a further aspect, the invention relates to a method of treating COVID-19, particularly Severe COVID-19 in a subject in need thereof, comprising administering to the subject a therapeutically effective amount of an antagonist of CD44/Hyaluronic Acid (HA) pathway.

In some embodiments, the antagonist of CD44/Hyaluronic Acid (HA) pathway of the invention is administered in combination with anti-Cytokine Release Syndrome (CRS) treatment.

In some embodiments, the antagonist of CD44/Hyaluronic Acid (HA) pathway of the invention is administered in combination with anti-severe COVID-19-related CRS treatment.

In some embodiments, the antagonist of CD44/Hyaluronic Acid (HA) pathway of the invention is administered in combination with any further compound which is used in the treatment of Respiratory Distress Syndrome (ARDS), Macrophage Activation Syndrome (MAS), Iron related inflammatory diseases and/or Alveolar inflammatory responses.

In some embodiments, the antagonist of CD44/Hyaluronic Acid (HA) pathway of the invention is administered in combination with anti-COVID-19 treatment, particularly anti-Severe COVID-19 treatment.

In particular, the antagonist of CD44/Hyaluronic Acid (HA) pathway of the invention may be administered in combination with anti-inflammatory agents.

The term "anti-Cytokine Release Syndrome (CRS) treatment" has its general meaning in the art and refers to any compound which is used in the treatment of CRS and any type of CRS therapy undergone by the CRS subjects including any compound which is used in the treatment of CRS (Shimabukuro-Vornhagen et al., Cytokine release syndrome. J Immunother Cancer. 2018 Jun 15;6(1):56. doi: 10.1186/s40425-018-0343-9; Moore JB, June CH. Cytokine release syndrome in severe COVID-19. Science. 2020 May 1;368(6490):473-474. doi: 10.1126/science.abb8925).

Compounds and therapies used in the treatment of CRS include, but are not limited to antihistamines; antipyretics; oral or intravenous fluids used in rehydration therapy; antibiotic therapy; BiTE blinatumomab; II,6 antagonists such as agents targeting IL6 such as Siltuximab or agents targeting II,6R such as tocilizumab and sarilumab; anti-TNFα agents such as etanercept; anti-GM-CSF agents such as Lenzilumab; ibrutinib; and T cell-depleting antibody therapies such as alemtuzumab and antithymocyte globulin (ATG), II,-1R-based inhibitors (anakinra) and cyclophosphamide; and anti-inflammatory agents.

The term "anti-COVID-19 treatment" has its general meaning in the art and refers to any type of COVID-19 therapy undergone by the COVID-19 subjects including any compound which is used in the treatment of COVID-19 such as anti-viral agents, compounds used in the treatment of severe COVID-19-related CRS and anti-inflammatory agents (Zhou et al., M, Zhang X, Qu J. Coronavirus disease 2019 (COVID-19): a clinical update. Front Med. 2020 Apr; 14(2): 126-135. doi: 10.1007/s11684-020-0767-8).

Compounds and therapies used in the treatment of COVID-19 include, but are not limited to anti-viral agents such as remdesivir (GS-5734), GS-441524, lopinavir, ritonavir, lopinavir/ritonavir (LPV/r) combination, lopinavir/ritonavir and interferon beta 1-alpha combination (LPV/r/INF-β-1a combination), ribavirin, ribavirin and interferon-α combination (ribavirin and INF-α2b combination) and abidol (umifenovir or Arbidol); IL6 antagonists such as agents targeting IL6 such as Siltuximab, or agents targeting IL6R such as tocilizumab, sarilumab; IL-1 antagonists such as antagonist of IL-1 β anakinra; TNF blockers; IFN- αβ inhibitors; chloroquine; hydroxychloroquine (HCQ); corticosteroids; antimicrobial agents such as antibiotics and antifungal agents; intravenous immunoglobulin (IVIG); convalescent plasma therapy; anticoagulant agents; oxygen therapy such as high-flow nasal cannula oxygen therapy (HFNC), noninvasive mechanical ventilation (NIV), invasive mechanical ventilation and extracorporeal membrane oxygenation (ECMO); continuous renal replacement therapy (CRRT); and compounds used in the treatment of CRS.

Anti-inflammatory agents include, but are not limited to nonsteroidal anti-inflammatory drugs (NSAID) such as indomethacin, ibuprofen, salicylic acid acetate, sulindac, piroxicam, and naproxen; and corticosteroids.

### Pharmaceutical composition

The compounds of the invention may be used or prepared in a pharmaceutical composition.

In one embodiment, the invention relates to a pharmaceutical composition comprising the compound of the invention and a pharmaceutical acceptable carrier for use in the treatment of Cytokine Release Syndrome (CRS) in a subject of need thereof.

In some embodiments, the invention relates to a pharmaceutical composition comprising the compound of the invention and a pharmaceutical acceptable carrier for use in the treatment of severe COVID-19-related CRS in a subject in need thereof.

Typically, the compound of the invention may be combined with pharmaceutically acceptable excipients, and optionally sustained-release matrices, such as biodegradable polymers, to form therapeutic compositions.

Typically the compounds according to the invention as described above are administered to the patient in a therapeutically effective amount.

By a "therapeutically effective amount" of the compound of the present invention as above described is meant a sufficient amount of the compound at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific compound employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the compound of the present invention for the symptomatic adjustment of the dosage to the patient to be treated. A medicament typically contains from about 0.01 mg to about 500 mg of the compound of the present invention, preferably from 1 mg to about 100 mg of the compound of the present invention. An effective amount of the drug is ordinarily supplied at a dosage level from 0.0002 mg/kg to about 20 mg/kg of body weight per day, especially from about 0.001 mg/kg to 7 mg/kg of body weight per day.

In a particular embodiment, the compound according to the invention may be used in a concentration between 0.01 µM and 20 µM, particularly, the compound of the invention may be used in a concentration of 0.01, 0.05, 0.1, 0.5, 1.0, 2.5, 5.0, 10.0, 15.0, 20.0 µM.

According to the invention, the compound of the present invention is administered to the subject in the form of a pharmaceutical composition. Typically, the compound of the present invention may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Typically, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The compound of the present invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized agents of the present inventions into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the compound of the present invention plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

Pharmaceutical compositions of the invention may include any further compound which is used in the treatment of Cytokine Release Syndrome (CRS).

In some embodiments, the pharmaceutical compositions of the invention may include any further compound which is used in the treatment of severe COVID-19-related CRS.

In some embodiments, the pharmaceutical compositions of the invention may include any further compound which is used in the treatment of Respiratory Distress Syndrome (ARDS), Macrophage Activation Syndrome (MAS), Iron related inflammatory diseases and/or Alveolar inflammatory responses.

In some embodiments, the pharmaceutical compositions of the invention may include any further compound which is used in the treatment of COVID-19, particularly Severe COVID-19.

In one embodiment, said additional active compounds may be contained in the same composition or administrated separately.

In another embodiment, the pharmaceutical composition of the invention relates to combined preparation for simultaneous, separate or sequential use in the treatment of Cytokine Release Syndrome (CRS) in a subject in need thereof.

In some embodiments, the pharmaceutical composition of the invention relates to combined preparation for simultaneous, separate or sequential use in the treatment of severe COVID-19-related CRS in a subject in need thereof.

In another embodiment, the pharmaceutical composition of the invention relates to combined preparation for simultaneous, separate or sequential use in the treatment of Respiratory Distress Syndrome (ARDS), Macrophage Activation Syndrome (MAS), Iron related inflammatory diseases and/or Alveolar inflammatory responses.

In another embodiment, the pharmaceutical composition of the invention relates to combined preparation for simultaneous, separate or sequential use in the treatment of COVID-19, particularly Severe COVID-19.

The invention also provides kits comprising the compound of the invention. Kits containing the compound of the invention find use in therapeutic methods.

### Diagnostic method

In a further aspect, the invention relates to the use of CD44 as biomarker of predisposition to severe COVID-19, and then predicting the occurrence of a severe COVID-19.

Accordingly, the invention relates to a method of identifying a subject having or at risk of having or developing severe COVID-19, comprising a step of measuring in a biological sample obtained from said subject the expression level of CD44.

As used herein, the term "biological sample" refers to any biological sample derived from the subject such as blood sample, peripheral blood sample, bronchoalveolar sample, bronchial sample, alveolar sample, nasopharyngeal sample, sputum sample, mucus sample, trachea sample or nasal sample.

The method of the invention may further comprise a step consisting of comparing the expression level of CD44 in the biological sample with a reference value, wherein detecting differential in the expression level of CD44 between the biological sample and the reference value is indicative of subject having or at risk of having or developing Severe COVID-19.

As used herein, the "reference value" refers to a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Preferably, the person skilled in the art may compare the expression level (obtained according to the method of the invention) with a defined threshold value. In one embodiment of the present invention, the threshold value is derived from the expression level (or ratio, or score) determined in a biological sample derived from one or more subjects having severe COVID-19. Furthermore, retrospective measurement of the expression level (or ratio, or scores) in properly banked historical subject samples may be used in establishing these threshold values.

In one embodiment, the reference value may correspond to the expression level of CD44 determined in a biological sample associated with a subject not having or not at risk of having or developing Severe COVID-19. Accordingly, a higher expression level of CD44 than the reference value is indicative of a subject having or at risk of having or developing Severe COVID-19, and a lower or equal expression level of CD44 than the reference value is indicative of a subject not having or not at risk of having or developing Severe COVID-19.

In another embodiment, the reference value may correspond to the expression level of CD44 determined in a biological sample associated with a subject having or at risk of having or developing with Severe COVID-19. Accordingly, a higher or equal expression level of CD44 than the reference value is indicative of a subject having or at risk of having or developing Severe COVID-19, and a lower expression level of CD44 than the reference value is indicative of a subject not having or not at risk of having or developing Severe COVID-19.

Analyzing the expression level of CD44 may be assessed by any of a wide variety of well-known methods for detecting expression of a transcribed nucleic acid or translated protein.

In one embodiment, the expression level of CD44 is assessed by analyzing the expression of mRNA transcript or mRNA precursors, such as nascent RNA, of CD44 gene. Said analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TaqMan), and probes arrays such as GeneChip(TM) DNA Arrays (AFFYMETRIX).

Advantageously, the analysis of the expression level of mRNA transcribed from the gene encoding for CD44 involves the process of nucleic acid amplification, e. g., by RT-PCR (the experimental embodiment set forth in U. S. Patent No. 4,683, 202), ligase chain reaction (Barany, 1991), self sustained sequence replication (Guatelli et al., 1990), transcriptional amplification system (Kwoh et al., 1989), Q-Beta Replicase (Lizardi et al., 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another embodiment, the expression level of CD44 is assessed by analyzing the expression of the protein translated from said gene. Said analysis can be assessed using an antibody (e.g., a radio-labeled, chromophore- labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.), which binds specifically to the protein translated from the gene encoding for CD44.

Said analysis can be assessed by a variety of techniques well known from one of skill in the art including, but not limited to, enzyme immunoassay (EIA), radioimmunoassay (RIA), Western blot analysis and enzyme linked immunoabsorbant assay (RIA).

In a further aspect, the method of the invention is performed by identifying macrophage activation.

In one embodiment, the macrophage activation is assessed by measuring the cytokine profile. Analyzing the macrophage activation may be assessed according to the method of the invention.

A further aspect of the invention relates to a method of monitoring COVID-19 progression by performing the method of the invention.

In one embodiment, the present invention relates to a method of treating Severe COVID-19 in a subject in need thereof comprising the steps of:
(i) identifying a subject having or at risk of having or developing a Severe COVID-19 by performing the method according to the invention, and
(ii) administering to said subject an antagonist of CD44/Hyaluronic Acid (HA) pathway when it is concluded that the subject has or is at risk of having or developing Severe COVID-19.

In a further aspect, the invention relates to the use of CD44 as biomarker of efficiency of the compound of the invention, and then predicting the response of a subject to the compound of the invention.

Accordingly, the invention relates to a method for determining whether a subject suffering from Cytokine Release Syndrome (CRS) will achieve a response with an antagonist of CD44/Hyaluronic Acid (HA) pathway comprising determining the expression level of CD44, wherein said expression level correlates with the response of the subject to the treatment.

In another aspect, the invention relates to a method for determining whether a subject suffering from COVID-19, particularly severe COVID-19 will achieve a response with antagonist of CD44/Hyaluronic Acid (HA) pathway comprising determining the expression level of CD44, wherein said expression level correlates with the response of the subject to the treatment.

In some embodiments, the method comprises the steps of i) determining the expression level of CD44, ii) comparing the expression level determined at step i) with a predetermined reference value and iii) concluding that the subject will achieve a response when the level determined at step i) is higher than the predetermined reference value.

The method is thus particularly suitable for discriminating responder from non responder. As used herein the term "responder" in the context of the present disclosure refers to a subject that will achieve a response, i.e. a subject where the disease is eradicated, reduced or improved. According to the invention, the responders have an objective response and therefore the term does not encompass subjects having a stabilized disease such that the disease is not progressing after the therapy. A non-responder or refractory subject includes subjects for whom the disease does not show reduction or improvement after the therapy. According to the invention the term "non responder" also includes subjects having a stabilized disease. Typically, the characterization of the subject as a responder or non-responder can be performed by reference to a standard or a training set. The standard may be the profile of a subject who is known to be a responder or non responder or alternatively may be a numerical value. Such predetermined standards may be provided in any suitable form, such as a printed list or diagram, computer software program, or other media. When it is concluded that the subject is a non-responder, the physician could take the decision to stop and change the therapy to avoid any further adverse sides effects.

In one embodiment, the present invention relates to a method of treating Severe COVID-19 in a subject in need thereof comprising the steps of:
(i) determining whether the subject will achieve a response with antagonist of CD44/Hyaluronic Acid (HA) pathway by performing the method according to the invention, and
(ii) administering to said subject an antagonist of CD44/Hyaluronic Acid (HA) pathway when it is concluded that the subject is responder.

The invention will be further illustrated by the following figures and examples. However, these examples and figures should not be interpreted in any way as limiting the scope of the present invention.

### FIGURES:

Figure 1: Increase of CD44 and RhoNOX-M in activated macrophages. GM-CSF-treated monocytes (M1) were activated with LPS (100 ng/mL) during 24 hours and CSF1-treated monocytes were activated with IL4 (20 ng/mL) during 24 hours. CD44, TfR and RhoNOX-M were measured by flow cytometry.
**Figure 2****: CD44-dependent iron endocytosis during macrophagic activation**
**Figure 3****: CD44 antagonists induce decreased levels of cellular iron in activated monocyte-derived macrophages.** (**A**) ICP-MS of cellular iron and copper in activated (act.) monocyte-derived macrophages (MDM) treated with siCtrl. or siCD44. n=7 donors. (**B**) Representative western blot of CD44 in act. MDM transfected with siRNA against CD44 or control siRNA. n=4 donors. Mann-Whitney test. Mean values ± SEM. (**C**) ICP-MS of cellular iron and copper in act. MDM treated with the anti-CD44 antibody RG7356. n=7 donors. Different donors are denoted in each panel with distinctly dots.

### EXAMPLE:

### EXAMPLE 1

### Material & Methods

**Chemical reagents and antibodies.** GM-CSF and IFN-gamma were obtained from Miltenyi (Miltenyi Biotec, Somerville, MA, USA), Deferoxamine (DFO) from Sigma-Aldrich (SIGMA-ALDRICH, Saint-Quentin Fallavier, France), RhoNox-M (in-house), Hyaluronate Fluorescein (FITC-Hyal) (#YH4532, Carbosynth, San Diego, CA, USA), Transferrin from human serum, Alexa Fluor 647 conjugate (TF-647) (#T23366) (ThermoFisher Scientific, Waltham, MA, USA). We used antibodies to CD44 (#ab189524, WB: 1:30000, Abcam, Cambridge, MA, USA), Ferritin (#ab75973, WB: 1:1000, Abcam), H3K9me2 (#4658S, WB: 1:1000, Cell Signaling, Danvers, MA, USA), Transferrin receptor 1 (TfR1) (#13-6800, WB: 1:1000, Thermo Fisher Scientific).

**Cell culture.** Peripheral blood samples were collected from healthy donors. Pan monocytes were sorted using microbeads according to the manufacturer's instructions (Miltenyi Biotec, Somerville, MA, USA), cultured in RPMI 1640 with glutamine (Thermo Fisher Scientific) supplemented with 10% fetal bovine serum and exposed to 100 ng/mL GM-CSF to generate their differentiation. After 5 days of differentiation, macrophages were activated with IFN-g (50 ng/mL) and treated by DF0 (10 µm) during 24 hours.

**Bronchoalveolar lavages (BAL).** BAL from COVID patients were centrifuged, then alveolar macrophages were purified by adherence in plastic dishes in serum-free RPMI 1640 culture medium without serum or antibiotics at 37°C in an humidified atmosphere containing 5% CO2. After approximatively 15-30 min of incubation the nonadherent cells were removed and the layer of adherent cells were washed twice with 10 mL of ice-cold sterile PBS. The adherent cells were treated overnight by DFO or RG7356.

**Cell morphology.** Phase contrast images were captured with a CKX41 microscope (Olympus) and cell Sens Entry imaging software (Olympus) before manually tracing the long and short axes of each cell (long axis, longest length of the cell; short axis, length across the nucleus in a direction perpendicular to the long axis) to measure the elongation factor as the ratio of these axes.

**Flow cytometry analysis of cell phenotype.** Cells were washed with ice-cold PBS, incubated with Fc block (Human TruStain FcX, Biolegend, London, United Kingdom, 1/20) for 15 min, incubated with antibodies for 20 minutes at 4°C and washed before analysis using a BD LSRFortessa X-20. Antibodies were AlexaFluor700-CD80 (#561133, BD, Franklin Lakes, NJ, USA), PE/Cy7-CD86 (#561128, BD), APC/Alexa750-CD71 (#A89313, Beckman coulter, Brea, CA, USA), Krome orange-CD14 (#B01175, Beckman coulter), Pacific Blue-CD16 (#A82792, Beckman coulter), PE-CD163 (#556018, BD) and AlexaFluor647-CD44 (#NB500-481AF647, Novus Biologicals). The data were analyzed with FlowJo software v. 10.0.00003.

**Immunofluorescence microscopy.** Cells were spotted on slides using a cytospin centrifuge, washed in PBS, fixed with 4% formaldehyde in PBS for 20 minutes, washed with PBS, post-fixed and permeabilized with cold 70% ethanol for 20 minutes, washed with PBS, blocked with 8% bovine serum albumin (BSA) in PBS for 1 hour, incubated with the first antibody in 1% BSA in PBS for 2 hours, washed and incubated with a secondary antibody conjugated with Alexa Fluor 488 or 555 for 1 hour, washed and mounted by using Vectashield mounting medium with DAPI (Vector Laboratories, Burlingame, CA, USA).

Other fluorescence imaging experiments. Live cells were incubated for 1 hour at 37 °C with 5% CO2 in medium containing RhoNox-M, FITC-Hyal, TF-647. Then, cells were washed three times with PBS, spotted on slides using a cytospin centrifuge and mounted by using Vectashield mounting medium with DAPI (Vector Laboratories).

Fluorescence images were acquired using a Deltavision real-time microscope (Applied Precision). 40×/1.4NA, 60×/1.4NA and 100×/1.4NA objectives were used for 2D and 3D acquisitions. Data were deconvoluted with SoftWorx (Ratio conservative - 15 iterations, Applied Precision) and processed with ImageJ. All images were acquired as z-stacks.

**Inductively coupled plasma mass spectrometry.** Glass vials equipped with Teflon septa were cleaned with nitric acid 65% (VWR), washed with ultrapure water (Sigma-Aldrich) and dried. Cells were plated 24 h prior to the experiments. In all experiments, cells were incubated for 2 h with FBS-free medium prior to treatment. Cells were harvested, followed by two washes with PBS. Cells were then counted using an automated cell counter (Entek) and transferred in 100 µL PBS to clean vials, and samples were lyophilized using a freeze dryer (CHRIST). Samples were subsequently mixed with nitric acid 65% overnight followed by heating at 80 °C for 2 h. Samples were diluted with ultrapure water (Sigma-Aldrich) to a final concentration of 0.475 N nitric acid and transferred to metal-free centrifuge vials (VWR) for subsequent ICP-MS analysis. ⁵⁶Fe concentrations were measured using an Agilent 7900 ICP-QMS in low-resolution mode. Sample introduction was achieved with a micro-nebulizer (MicroMist, 0.2 mL/min) through a Scott spray chamber. Isotopes were measured using a collision-reaction interface with helium gas (5 mL/min) to remove polyatomic interferences. Scandium and indium internal standards were injected after inline mixing with the samples to control the absence of signal drift and matrix effects. A mix of certified standards was measured at concentrations spanning those of the samples to convert count measurements to concentrations in the solution. Uncertainties on sample concentrations were calculated using algebraic propagation of ICP-MS blank and sample counts uncertainties. Values were normalized by dry weight and cell number.

**Immunoblot analyses.** Cells were washed twice in PBS and lysed in 2× Laemmli buffer containing benzonase (#VWR, Fontenay-Sous-Bois, France), extracts were incubated at 37 °C for 1 h, and quantified using a NanoDrop 2000 spectrophotometer (ThermoFisher Scientific). Protein lysates were resolved on Nu-PAGE 4-12% Bis-Tris gels (Invitrogen, Carlsbad, CA, USA) and electroblotted onto nitrocellulose membranes (Bio-Rad, Hercules, CA, USA). Membranes saturated with milk were incubated overnight at 4 °C with primary antibodies, then for 45 min with peroxydase-conjugated anti-IgG secondary antibodies (Jackson Laboratories, Bar Harbor, ME, USA). Signals were revealed by autoradiography using the SuperSignal West Pico PLUS or West Femto enhanced chemiluminescent detection kits (Thermo Fisher Scientific).

**Cytokine profile in human macrophage supernatants.** IL1 beta, IL4, IL6, IL8, IL10, IL12, IFNgamma and TNF-alpha concentrations were measured in cell culture supernatants using the human Pro-Inflammatory Combo 1 U-Plex (MSD, Rockville, MD, US). The kit was run according to the manufacturer's guidelines and the chemiluminescence signal was measured on a Sector Imager 2400 (MSD).

**RNA sequencing.** RNA extracted using the RNeasy mini kit (QIAGEN, Germantown, MD, USA) was processed using SureSelect Automated Strand Specific RNA Library Preparation Kit and sequenced on Illumina Novaseq 6000 in paired-end 100bp mode in order to reach at least 40 million reads per sample. Gene expression validation will be done by qRT-PCR are listed below.

### Results

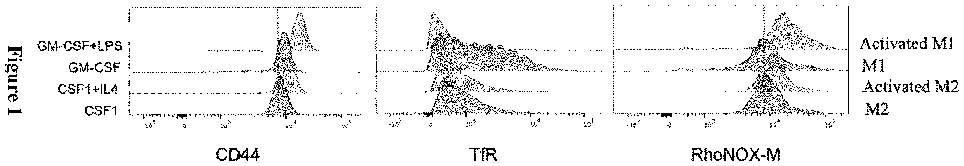

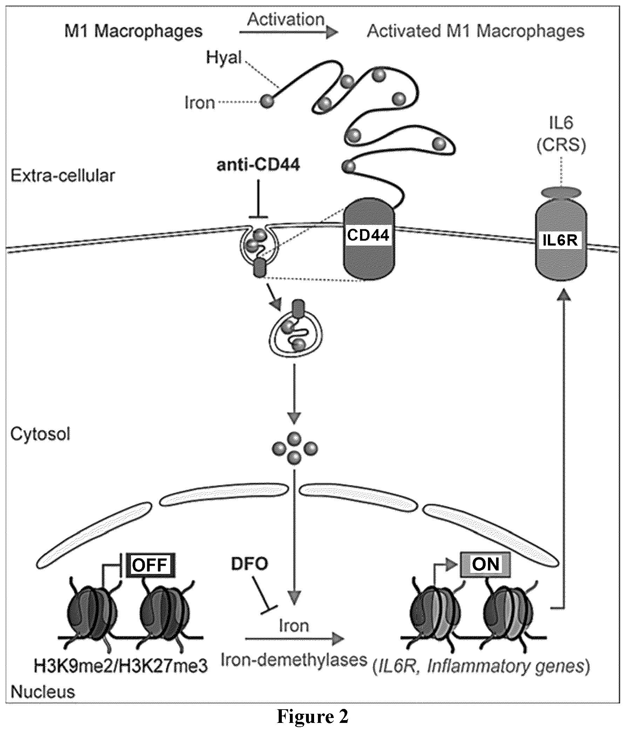

Our unpublished data indicate that during activation of M1 macrophages, iron endocytosis is upregulated in a CD44-dependent manner and that CD44 protein levels increase. Moreover, lysosomal Fe²⁺ is increased in activated M1 macrophages as observed by RhoNox-1. This effect is specific to M1 macrophages and levels of the canonical iron endocytosis protein TfR1/CD71 remain unchanged (Figures 1 and 2).

### EXAMPLE 2

### Material & Methods

**Cell culture.** Peripheral blood samples were collected from distinct healthy donors (Etablissement Français du Sang). Pan monocytes were isolated by negative magnetic sorting using microbeads according to the manufacturer's instructions (Miltenyi Biotec, 130-096-537), and cultured in RPMI 1640 supplemented with glutamine (Thermo Fisher Scientific, 61870010), 10% fetal bovine serum and treated with granulocyte-macrophage colony-stimulating factor (GM-CSF, Miltenyi Biotec, 130-093-866, 100 ng/mL) to induce differentiation into macrophages (MDM). At day 5 of differentiation, MDM were treated with lipopolysaccharides (LPS, InvivoGen, tlrl-3pelps, 100 ng/mL, 24 h) and interferon-γ (IFNγ, Miltenyi Biotec, 130-096-484, 20 ng/mL, 24 h) to generate activated MDM (act. MDM) and co-treated with anti-CD44 antibody RG7356 or transfected with a control or CD44 siRNA using the AMAXA Nucleofector transfection system (LONZA). Suitable small interfering RNAs for specific down-regulation of CD44 were the followings
5' -GAAUAUAACCUGCCGCUUU-3' (SEQ ID NO: 1),
5' -CAAGUGGACUCAACGGAGA-3' (SEQ ID NO: 2),
5' -CGAAGAAGGUGUGUGGGCAGA-3' (SEQ ID NO: 3), and
5' -GAUCAACAGUGGCAAUGGA-3' (SEQ ID NO: 4).

**Western Blotting.** Cells were treated as indicated and then washed with 1× PBS. Proteins were solubilized in 2× Laemmli buffer containing benzonase (VWR, 70664-3, 1:100), extracts were incubated at 37 °C for 1 h, and quantified using a NanoDrop 2000 spectrophotometer (ThermoFisher Scientific). Protein lysates were resolved by SDS-PAGE electrophoresis (Invitrogen sure-lock system and Nu-PAGE 4-12% Bis-Tris precast gels) and transferred onto nitrocellulose membranes (Amersham Protran 0.45 µm) using a Trans-Blot SD semi-dry electrophoretic transfer cell (Bio-rad). Membranes were blocked with 5% non-fat skimmed milk powder in 0.1% Tween-20/1× PBS for 1 h. Blots were then probed with the relevant primary antibodies in 5% BSA, 0.1% Tween-20/1× PBS at 4 °C overnight with gentle motion. Membranes were washed with 0.1% Tween-20/1× PBS three times and incubated with horseradish peroxidase conjugated secondary antibodies (Jackson Laboratories) in 5% non-fat skimmed milk powder, 0.1% Tween-20/1× PBS for 1 h at room temperature and washed three times with 0.1% Tween-20/1× PBS. Antigens were detected using the SuperSignal West Pico PLUS chemiluminescent detection kits (ThermoFisher Scientific, 34580 and 34096). Signals were recorded using a Fusion Solo S Imaging System (Vilber) and quantified with ImageJ using pixel intensity normalized against the signal of γ-tubulin.

**Inductively coupled plasma mass spectrometry (ICP-MS).** HA (Carbosynth, FH45321, 600-1000 kDa, 1 mg/mL) was added together with LPS and IFNγ and cells were treated for 24 h. Glass vials equipped with Teflon septa were cleaned with nitric acid 65% (VWR, Suprapur, 1.00441.0250), washed with ultrapure water (Sigma-Aldrich, 1012620500) and dried. Cells were harvested followed by two washes with 1× PBS. Cells were then counted using an automated cell counter (Entek) and transferred in 200 µL 1× PBS to the cleaned glass vials, and samples were lyophilized using a freeze dryer (CHRIST, 22080). Samples were subsequently mixed with nitric acid 65% overnight and heated at 80 °C for 2 h. Samples were diluted with ultrapure water to a final concentration of 0.475 N nitric acid and transferred to metal-free centrifuge vials (VWR, 89049-172) for subsequent ICP-MS analysis. Amounts of ⁵⁶Fe and ⁶³Cu were measured using an Agilent 7900 ICP-QMS in low-resolution mode. Sample introduction was achieved with a micro-nebulizer (MicroMist, 0.2 mL/min) through a Scott spray chamber. Isotopes were measured using a collision-reaction interface with helium gas (5 mL/min) to remove polyatomic interferences. Scandium and indium internal standards were injected after inline mixing with the samples to control the absence of signal drift and matrix effects. A mix of certified standards was measured at concentrations spanning those of the samples to convert count measurements to concentrations in the solution. Uncertainties on sample concentrations were calculated using algebraic propagation of ICP-MS blank and sample counts uncertainties. Values were normalized against cell number.

### Results

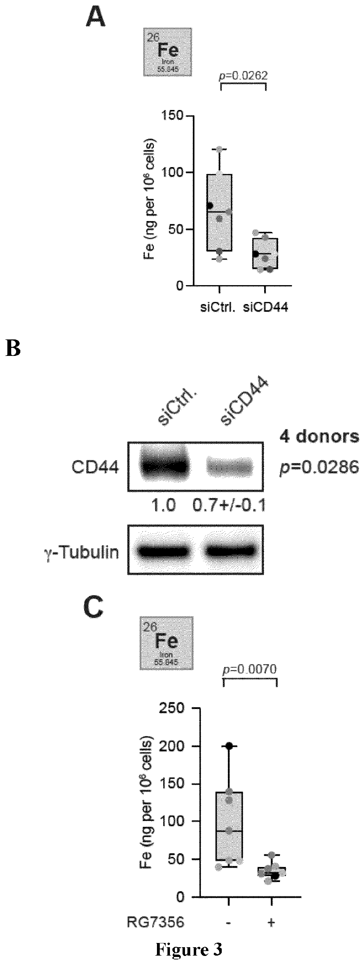

Down-regulation of CD44 using a small interfering RNA (siRNA) against CD44 reduced the total amount of iron in activated monocyte-derived M1 macrophages (act. MDM) compared to the control RNA (Figure 3A, B). In addition, treatment with a blocking antibody that binds CD44 (RG7356), reduces the amount of total iron in act MDM (Figure 3C). These data confirmed that CD44 mediates iron uptake during activation of inflammatory macrophages.

Altogether, these data are hinting towards a direct role of CD44-mediated iron endocytosis in the severe inflammatory response observed in SARS-CoV-2 patients. Institut Curie has considerable experience with the use of RG7356, a clinically approved anti-CD44 antibody, which has shown promising results in cancer patients with poor outcomes (First-in-human phase I clinical trial of RG7356, an anti-CD44 humanized antibody, in patients with advanced, CD44-expressing solid tumors. *Oncotarget* (2016), DOI: 10.18632/oncotarget.11098). Here, the inventors evaluate the effect of the use of antagonist of CD44/Hyaluronic Acid (HA) pathway and targeting of iron homeostasis such as by using DFO to antagonize IL6 signalling and to protect SARS-CoV-2 patients from CRS.

### REFERENCES:

Throughout this application, various references describe the state of the art to which this invention pertains. The disclosures of these references are hereby incorporated by reference into the present disclosure.

Crayne CB, Albeituni S, Nichols KE, Cron RQ. The Immunology of Macrophage Activation Syndrome. Front Immunol. 2019 Feb 1;10:119. doi:10.3389/fimmu.2019.00119.

D'Arena G, Calapai G, Deaglio S. Anti-CD44 mAb for the treatment of B-cell chronic lymphocytic leukemia and other hematological malignancies: evaluation of WO2013063498. Expert Opin Ther Pat. 2014 Jul;24(7):821-8. doi: 10.1517/13543776.2014.915942.

Davila ML, Riviere I, Wang X, Bartido S, Park J, Curran K, Chung SS, Stefanski J, Borquez-Ojeda O, Olszewska M, Qu J, Wasielewska T, He Q, Fink M, Shinglot H, Youssif M, Satter M, Wang Y, Hosey J, Quintanilla H, Halton E, Bernal Y, Bouhassira DC, Arcila ME, Gonen M, Roboz GJ, Maslak P, Douer D, Frattini MG, Giralt S, Sadelain M, Brentjens R. Efficacy and toxicity management of 19-28z CAR T cell therapy in B cell acute lymphoblastic leukemia. Sci Transl Med. 2014 Feb 19;6(224):224ra25. doi: 10.1126/scitranslmed.

Hernández-Beeftink T, Guillen-Guio B, Villar J, Flores C. Genomics and the Acute Respiratory Distress Syndrome: Current and Future Directions. Int J Mol Sci. 2019 Aug 16;20(16). pii: E4004. doi: 10.3390/ijms20164004.

Khan A, Singh P, Srivastava A. Synthesis, nature and utility of universal iron chelator - Siderophore: A review. Microbiol Res. 2018 Jul-Aug;212-213:103-111. doi: 10.1016/j.micres.2017.10.012.

Kontoghiorghes GJ. Comparative efficacy and toxicity of desferrioxamine, deferiprone and other iron and aluminium chelating drugs. Toxicol Lett. 1995 Oct;80(1-3):1-18.

Lerkvaleekul B, Vilaiyuk S. Macrophage activation syndrome: early diagnosis is key. Open Access Rheumatol. 2018 Aug 31; 10: 117-128. doi: 10.2147/OARRR.S151013.

Lima TG, Benevides FLN, Esmeraldo Filho FL, Farias IS, Dourado DXC, Fontenele EGP, Moraes MEA, Quidute ARP. Treatment of iron overload syndrome: a general review. Rev AssocMedBras (1992). 2019 Oct 10;65(9):1216-1222. doi:10.1590/1806-9282.65.9.1216.

Maude SL, Barrett D, Teachey DT, Grupp SA. Managing cytokine release syndrome associated with novel T cell-engaging therapies. Cancer J. 2014 Mar-Apr;20(2):119-22. doi: 10.1097/PPO.0000000000000035.

McKee CM, Penno MB, Cowman M, Burdick MD, Stricter RM, Bao C, Noble PW. Hyaluronan (HA) fragments induce chemokine gene expression in alveolar macrophages. The role of HA size and CD44. J Clin Invest. 1996 Nov 15;98(10):2403-13. DOI: 10.1172/JCI119054.

Menke-van der Houven van Oordt CW, Gomez-Roca C, van Herpen C, Coveler AL, Mahalingam D, Verheul HM, van der Graaf WT, Christen R, Rüttinger D, Weigand S, Cannarile MA, Heil F, Brewster M, Walz AC, Nayak TK, Guarin E, Meresse V, Le Tourneau C. First-in-human phase I clinical trial of RG7356, an anti-CD44 humanized antibody, in patients with advanced, CD44-expressing solid tumors. Oncotarget. 2016 Nov 29;7(48):80046-80058. doi: 10.18632/oncotarget.11098.

Merad M, Martin JC. Pathological inflammation in patients with COVID-19: a key role for monocytes and macrophages. Nat Rev Immunol. 2020 May 6. doi:10.1038/s41577-020-0331-4.

Mobarra N, Shanaki M, Ehteram H, Nasiri H, Sahmani M, Saeidi M, Goudarzi M, Pourkarim H, Azad M. A Review on Iron Chelators in Treatment of Iron Overload Syndromes. Int J Hematol Oncol Stem Cell Res. 2016 Oct 1;10(4):239-247.

Moore JB, June CH. Cytokine release syndrome in severe COVID-19. Science. 2020 May 1;368(6490):473-474. doi: 10.1126/science.abb8925.

Müller Sebastian, Sindikubwabo Fabien, Cañeque Tatiana, Lafon Anne, Versini Antoine, Lombard Bérangère, Loew Damarys, Durand Adeline, Vallot Céline, Baulande Sylvain, Servant Nicolas, Rodriguez Raphaël. CD44 regulates epigenetic plasticity by mediating iron endocytosis. bioRxiv 693424; doi: https://doi.org/10.1101/693424. Accepted in Nature Chemistry*.*

Niesler U, Palmer A, Radermacher P, Huber-Lang MS. Role of alveolar macrophages in the inflammatory response after trauma. Shock. 2014 Jul;42(1):3-10. doi: 10.1097/SHK.0000000000000167.

Ponta, H., Sherman, L. & Herrlich, P. A. CD44: from adhesion molecules to signalling regulators. Nat. Rev. Mol. Cell Biol. 4, 33-45, (2003).

Ranieri VM, Rubenfeld GD, Thompson BT, Ferguson ND, Caldwell E, Fan E, Camporota L, Slutsky AS. ARDS Definition Task Force. Acute respiratory distress syndrome: the Berlin Definition. JAMA. 2012 Jun 20;307(23):2526-33. doi:10.1001/jama.2012.5669.

Shalabi H, Hahn K, Fry TJ, Shah NN. Novel Designs of Early Phase Trials for Cancer Therapeutics. Chapter 12 - Cell-Based Therapies: A New Frontier of Personalized Medicine. Editor(s): Shivaani Kummar, Chris Takimoto. Academic Press, 2018, Pages 175-191, ISBN 9780128125120, https://doi.org/10.1016/B978-0-12-812512-0.00012-9.

Shimabukuro-Vornhagen A, Gödel P, Subklewe M, Stemmler HJ, Schlößer HA, Schlaak M, Kochanek M, Böll B, von Bergwelt-Baildon MS. Cytokine release syndrome. J Immunother Cancer. 2018 Jun 15;6(1):56. doi:10.1186/s40425-018-0343-9.

Stelzer G, Rosen N, Plaschkes I, Zimmerman S, Twik M, Fishilevich S, Stein TI, Nudel R, Lieder I, Mazor Y, Kaplan S, Dahary D, Warshawsky D, Guan-Golan Y, Kohn A, Rappaport N, Safran M, Lancet D. The GeneCards Suite: From Gene Data Mining to Disease Genome Sequence Analyses. Curr Protoc Bioinformatics. 2016 Jun 20;54:1.30.1-1.30.33. doi: 10.1002/cpbi.5.

UniProt Consortium. UniProt: a worldwide hub of protein knowledge. Nucleic Acids Res. 2019 Jan 8;47(D1):D506-D515. doi: 10.1093/nar/gky1049.

Weigand S, Herting F, Maisel D, Nopora A, Voss E, Schaab C, Klammer M, Tebbe A. Global quantitative phosphoproteome analysis of human tumor xenografts treated with a CD44 antagonist. Cancer Res. 2012 Sep 1;72(17):4329-39. doi: 10.1158/0008-5472.CAN-12-0136.

Whyte CS, Morrow GB, Mitchell JL, Chowdary P, Mutch NJ. Fibrinolytic abnormalities in acute respiratory distress syndrome (ARDS) and versatility of thrombolytic drugs to treat COVID-19. J Thromb Haemost. 2020 Apr 23. doi:10.1111/jth.14872.

Zhou F, Yu T, Du R, Fan G, Liu Y, Liu Z, Xiang J, Wang Y, Song B, Gu X, Guan L, Wei Y, Li H, Wu X, Xu J, Tu S, Zhang Y, Chen H, Cao B. Clinical course and risk factors for mortality of adult inpatients with COVID-19 in Wuhan, China: a retrospective cohort study. Lancet. 2020 Mar 28;395(10229):1054-1062. doi:10.1016/S0140-6736(20)30566-3. Erratum in: Lancet. 2020 Mar 28;395(10229):1038. Lancet. 2020 Mar 28;395(10229):1038.

Zhou M, Zhang X, Qu J. Coronavirus disease 2019 (COVID-19): a clinical update. Front Med. 2020 Apr; 14(2):126-135. doi: 10.1007/s11684-020-0767-8.

Zöller, M. CD44: can a cancer-initiating cell profit from an abundantly expressed molecule? Nat. Rev. Cancer 11, 254-267, (2011).

## Claims

1. An antagonist of CD44/Hyaluronic Acid pathway for use in the treatment of cytokine release syndrome in a subject in need thereof, wherein said antagonist of CD44/Hyaluronic Acid (HA) pathway is selected from the group consisting of compound targeting CD44 selected from the group consisting of CD44 antagonists and CD44 expression inhibitors.

2. The antagonist of CD44/Hyaluronic Acid (HA) pathway for use according to claim 1, wherein the cytokine release syndrome is severe COVID-19-related cytokine release syndrome.

3. The antagonist of CD44/Hyaluronic Acid (HA) pathway for use according to any one of claims 1-2, wherein said CD44 antagonist is selected from the group consisting of small organic molecule, polypeptide, aptamer or antibody.

4. The antagonist of CD44/Hyaluronic Acid (HA) pathway for use according to claim 3, wherein said antibody is selected from the group consisting of RG7356 and Bivatuzumab.

5. The antagonist of CD44/Hyaluronic Acid (HA) pathway for use according to any one of claims 1-3, wherein said CD44 expression inhibitor is selected from the group consisting of antisense oligonucleotide, shRNA, siRNA, RNAi, and ribozyme.

6. The antagonist of CD44/Hyaluronic Acid (HA) pathway for use according to any one of claims 1-5, for use in the treatment of Respiratory Distress Syndrome (ARDS) in a subject in need thereof.

7. The antagonist of CD44/Hyaluronic Acid (HA) pathway for use according to any one of claims 1-5, for use in the treatment of Macrophage Activation Syndrome (MAS) in a subject in need thereof.

8. The antagonist of CD44/Hyaluronic Acid (HA) pathway for use according to any one of claims 1-5, for use in the treatment of Iron related inflammatory diseases and Alveolar inflammatory responses in a subject in need thereof.

## Patentansprüche

1. Ein Antagonist des CD44/Hyaluronsäure Wegs zur Verwendung in der Behandlung des Zytokinfreisetzungssyndroms in einem Subjekt, das das benötigt, wobei der Antagonist des CD44/Hyaluronsäure (HA) Wegs ausgewählt ist aus der Gruppe bestehend aus einer auf CD44-abzielende Verbindung ausgewählt aus der Gruppe bestehend aus CD44-Antagonsiten und CD44-Expressionsinhibitoren.

2. Der Antagonist des CD44/Hyaluronsäure Wegs zur Verwendung nach Anspruch 1, wobei das Zytokinfreisetzungssyndrom schweres Covid-19-bedingtes Zytokinfreisetzungssyndrom ist.

3. Der Antagonist des CD44/Hyaluronsäure Wegs zur Verwendung nach einem der Ansprüche 1-2, wobei der CD44-Antagonist ausgewählt ist aus der Gruppe bestehend aus kleinem organischen Molekül, Polypeptid, Aptamer oder Antikörper.

4. Der Antagonist des CD44/Hyaluronsäure Wegs zur Verwendung nach Anspruch 3, wobei der Antikörper ausgewählt ist aus der Gruppe bestehend aus RG7356 und Bivatuzumab.

5. Der Antagonist des CD44/Hyaluronsäure Wegs zur Verwendung nach einem der Ansprüche 1-3, wobei der CD44-Expressionsinhibitor ausgewählt ist aus der Gruppe bestehend aus Antisense-Oligonukleotid, shRNA, siRNA, RNAi und Ribozym.

6. Der Antagonist des CD44/Hyaluronsäure Wegs zur Verwendung nach einem der Ansprüche 1-5, zur Verwendung in der Behandlung von Atemnotsyndrom (ARDS) in einem Subjekt, das dies benötigt.

7. Der Antagonist des CD44/Hyaluronsäure Wegs zur Verwendung nach einem der Ansprüche 1-5, zur Verwendung in der Behandlung von Makrophagenaktivierungssyndrom (MAS) in einem Subjekt, das dies benötigt.

8. Der Antagonist des CD44/Hyaluronsäure Wegs zur Verwendung nach einem der Ansprüche 1-5, zur Verwendung in der Behandlung von Eisen-bedingten entzündlichen Erkrankungen und alveoläre Entzündungsreaktionen in einem Subjekt, das dies benötigt.

## Revendications

1. Antagoniste de la voie de CD44/acide hyaluronique pour une utilisation dans le traitement du syndrome de libération de cytokines chez un sujet en ayant besoin, dans lequel ledit antagoniste de la voie de CD44/acide hyaluronique (HA) est choisi dans le groupe constitué par un composé ciblant CD44 choisi dans le groupe constitué par les antagonistes de CD44 et les inhibiteurs de l'expression de CD44.

2. Antagoniste de la voie de CD44/acide hyaluronique (HA) pour une utilisation selon la revendication 1, dans lequel le syndrome de libération de cytokines est un syndrome de libération de cytokines associé à une COVID-19 sévère.

3. Antagoniste de la voie de CD44/acide hyaluronique (HA) pour une utilisation selon l'une quelconque des revendications 1 à 2, dans lequel ledit antagoniste de CD44 est choisi dans le groupe constitué par une petite molécule organique, un polypeptide, un aptamère, ou un anticorps.

4. Antagoniste de la voie de CD44/acide hyaluronique (HA) pour une utilisation selon la revendication 3, dans lequel ledit anticorps est choisi dans le groupe constitué par RG7356 et le bivatuzumab.

5. Antagoniste de la voie de CD44/acide hyaluronique (HA) pour une utilisation selon l'une quelconque des revendications 1 à 3, dans lequel ledit inhibiteur de l'expression de CD44 est choisi dans le groupe constitué par un oligonucléotide antisens, un ARNsh, un ARNsi, un ARNi, et un ribozyme.

6. Antagoniste de la voie de CD44/acide hyaluronique (HA) pour une utilisation selon l'une quelconque des revendications 1 à 5, pour une utilisation dans le traitement du syndrome de détresse respiratoire (SDRA) chez un sujet en ayant besoin.

7. Antagoniste de la voie de CD44/acide hyaluronique (HA) pour une utilisation selon l'une quelconque des revendications 1 à 5, pour une utilisation dans le traitement du syndrome d'activation macrophagique (SAM) chez un sujet en ayant besoin.

8. Antagoniste de la voie de CD44/acide hyaluronique (HA) pour une utilisation selon l'une quelconque des revendications 1 à 5, pour une utilisation dans le traitement de maladies inflammatoires associées au fer et de réponses inflammatoires alvéolaires chez un sujet en ayant besoin.
